# EUROPEAN PATENT SPECIFICATION

(11) **EP 2 189 957 B1**
(45) Date of publication and mention of the grant of the patent: **06.03.2013**
(21) Application number: 08169867.2
(22) Date of filing: 25.11.2008
(51) Int. Cl.: G08B 21/02, G08B 21/22, G08B 13/193, G08B 21/04

(54) **An apparatus with an infrared sensor and magnetic near field communication properties for monitoring activity in a selected area**
Vorrichtung mit einem Infrarotsensor und Eigenschaften zur Kommunikation mit magnetischem Nahfeld zur Überwachung von Aktivität in einem gewählten Bereich
Appareil doté d'un capteur à infrarouges et de propriétés de communication de champ magnétique proche pour surveiller l'activité dans une région sélectionnée

(43) Date of publication of application: 26.05.2010
(73) Proprietor: Yes-Group ApS, 8200 Århus N (DK)
(72) Inventor: Jensen, Kurt Holdgaard, 8230 Åbyhøj (DK)
(74) Representative: Medenwaldt, Robin

(56) References cited:
- US-A- 5 218 344
- US-A1- 2004 183 684
- US-B1- 6 661 343
- GUEULLE P: "UN DETECTEUR D'INACTIVITE A INFRAROUGE PASSIF" ELECTRONIQUE RADIO PLANS, SPE, PARIS, FR, no. 519, 1 February 1991 (1991-02-01), page 50,55/56, XP000200420 ISSN: 1144-5742

## Description

### Field of the Invention

The present invention relates to the use of an electronic communication apparatus according to claim 1.

### Background of the Invention

In contrast to most wireless communication systems, where a radio frequency wave propagates through free space, a wireless non-propagating magnetic field can be provided which is localized relatively close to the antenna due to its rapid power decrease with distance from the antenna. This localized confinement is due to the fact that the decrease of the near magnetic field is proportional to the sixth power of the distance, whereas the propagating radio frequency wave power rolls off with the square of the distance. Due to its confinement in space near the radiation source, magnetic near field is generally used for communication between closely spaced electronic devices, for example between mobile telephones and headphones or microphones.

Applications have also found way into the medical sector, where near field magnetic communication is used between an implant and an external device as disclosed in US patent application No. 2005/267550. For example, the implant may be an insulin pump with a glucose sensor and the external device a control apparatus for the pump with an alarm function to alert the patient in case of alarm conditions.

Magnetic near field sensors are generally known for identifying a person's allowance for access to restricted areas. For example, in US patent No. 6,700,544 by Anderson, a system is disclosed in which a magnetic near field scanning system communicates with an ID card of a person in order to decide on the allowance for passing a gate. In case that a person is not allowed to pass, an alarm may sound or the passage is prevented.

In hospitals, but also in elderly people's homes, a trend has emerged for monitoring the activity pattern of elderly people, for example as disclosed in the article "Monitoring daily living activities of elderly people in a nursing home using an infrared motion-detection system" by Suzuki et al., published in Telemedicine and e-Health. Apr 2006, Vol. 12, No. 2: 146-155.

A telephone alarm system with a special infrared detector using a Fresnel lense is disclosed in the article "Un détecteur d'inactiveité à infrarouge passif" by Patrick Gueulle published in Electronic Radio Plans (1991) Fevrier, No. 519, Paris, Fr.

Also, in hospitals, efforts are going on to register information about patients and visitors entering the hospital. A hospital movement tracking system has been described on the Internet site
http://www.dsta.gov.sg/index.php?option=com_content&task=view&id=2636&Itemid =401
where a department in a hospital issued a radio frequency identification device (a sensor card about the size of an identity card), in exchange for visitors identity cards and contact numbers at the registration counter. Also, the hospital staff carried permanent sensor cards. Each card was encoded with a unique identity and was associated with a person's identity card number. As a person moved around in the department, the radio frequency energy emitted from the sensor cards was detected by receivers installed in the ceiling. Information on when a person entered or left a certain area was captured and stored in the system.

US patent No. US6661343 discloses a system for motion detection, where an infrared motion detector upon detection of motion in the vicinity of the detector transmits a query signal and yields an alarm signal, if no reply signal is received from a corresponding transponder worn by an authorised person.

US5218344 discloses a personnel monitoring system, for example at a hospital, wherein a computer causes stationary transceivers to broadcast interrogation signals which are responded to by corresponding portable unit. The preferred signal type is electromagnetic.

US2004/0183684 discloses an alarm system for patients, where a transmitter is sending an alarm signal to a nurse's station in case that a button is pushed by the patient or a rotary switch is activated, for example by infants during turning movement or by bed-confined patients.

All these systems suffer from the fact of going against the trend of reducing the radiation in hospitals and homes, as there is a belief that the increased long term radiation background is a hazard for humans.

Another problem is the fact that the use of wireless devices in hospitals are so extensive that system break-down is frequent for several minutes every day. Such break-down is unacceptable for the safety of patients and personnel.

Thus, there is a need for an improved system.

### Object of the Invention

It is an object of the invention to provide a surveillance system for hospitals care service implying a reduced radiation level.

### Description of the Invention

This object is achieved with the use of an electronic communication apparatus according to claim 1.

The electronic communication apparatus according to the invention can be used in a hospital for checking whether persons in a patient's room are authorised to be there. If the infrared radiation from such a person is detected in a room, the apparatus sends a request signal for identification. If the person moving in the room is the patient itself and the patient is carrying a correspondingly configured badge or other corresponding responsive portable device, the device will send a response signal to the apparatus with the ID of the device.

Also, if hospital personnel have entered a room, the apparatus will send a request signal and receive a response signal from a corresponding portable device, for example badge or cell phone, of the personnel.

Authorised visitors for the patient may upon entrance of the hospital be equipped with a corresponding portable device identifying them as authorised visitors. As a consequence of the ID checking facility of the apparatus, it may also serve as a burglar alarm. The system is useful for checking that no unauthorised personnel or visitors are entering the hospital room of a patient.

The system is also useful in elderly people's private home, where the apparatus may check whether visitors, such as medical control personnel, helpers and/or relatives, can be correctly identified.

Alternative uses of such a system night be production facilities, research and development departments, banks, and other types of areas with restricted access, where control is necessary to assure that only authorized people have access. The apparatus may also be used as a door opening facility, for example as an extension of the system as disclosed in US patent No. 6,700,544 by Anderson.

The apparatus may advantageously be part of a control system for people suffering from dementia. In this case, the person may be equipped with a portable device, for example in the form of a badge. When the person enters or tries to enter a restricted area, for example tries to leave the hospital, the apparatus may induce an alarm or simply denying access through the door.

The apparatus utilizes the interplay between infrared signal for detection and magnetic near field radiation for communication. The magnetic near field radiation traverses the clothing of the personnel such that the portable device can be worn inside the clothing, which would be a problem, if the communication were with infrared radiation. Furthermore, in certain cases, it is an advantage that the near field radiation is directional, whereas infrared radiation usually is reflected from surfaces of furnitures, floors and walls.

In order to also save power, the apparatus may be configured such that control unit is programmed to minimize the power consumption of the electronic communication unit including causing a sleep mode of the transceiver when no activity signal is received from the infrared sensor. In addition, the control unit is programmed to change the sleep mode into an activity mode for a period of time as a response to the activity signal from the infrared sensor.

When the apparatus has received a response signal from the portable device, the consecutive action of the apparatus may be of different nature depending on the configuration and programming of the control unit.

In a first embodiment, the control unit is configured, upon receiving the ID of the portable device by the transceiver, to forward the ID of the portable electronic device as a digital signal to a central computer. Thus all received IDs are forwarded as a digital signal to the central computer. The central computer has means for identifying the sending apparatus, for example because the forward signal contains an ID of the apparatus. The central computer receiving the forwarded signal may then be programmed to store the received data and to run a check routine in which it is decided whether the response ID is authorised for being in the vicinity of the apparatus. If it turns out that the ID is not allowed in the selected area, an alarm routine may be initiated. For example, if the response ID is registered as being associated with another patient in the hospital, the patient not having authorisation to be in the selected area, the central computer may run a call routine for calling hospital personnel to take action, for example by stating the cause, the floor number and room number for the apparatus in question.

In another embodiment, the control unit is provided with an ID list and is configured to compare the received response ID with the ID list. Only if the ID of the portable electronic device is not found on the list, an alarm or the ID itself or both are transmitted as a digital signal to a central computer. In this case, the control check is made in the apparatus and not in the central computer. The advantage is that the data transfer between the apparatus and the central computer is minimised.

The connection between the apparatus and the central computer may be wired or wireless depending on the requirements in the facility, where the apparatus is placed. For example, if the apparatus is part in a hospital, a wired connection may be preferred in order to minimise radiation background in the hospital. On the other hand, if the apparatus is part of a person's home, a wireless telephone connection may be preferred in order to minimise initial costs for establishment of a security and surveillance system.

In an even further embodiment, the control unit is provided with an ID list and is configured to compare the received response ID with the ID list. Only if the ID of the portable electronic device is not found on the list, an alarm is sent to a predetermined terminal, for example a cell phone belonging to special personnel that may take action. The alarm may also be sent to neighbours or nearby located relatives such that help can be achieved relatively quickly.

In case of a wireless connection between the apparatus and the central computer or between the apparatus and the predetermined terminal, it should be noted that the apparatus has a short range magnetic near field transceiver, on the one hand, and a long range wireless transmitter or transceiver, on the other hand. Whereas the short range signals are used any time a motion is detected by the infrared sensor, the long range signal may be restricted to alarm situations, which reduces the overall long range radiation background.

If after a request signal, no response signal with an ID is received, the control unit is configured to automatically submit a digital alarm signal to a central computer or to a predetermined terminal. The lack of response ID indicates that unauthorised persons are in the selected area, and appropriate action should follow.

The apparatus according to the invention may also be used for checking for activity. The apparatus may be used to check whether a person in questions is entering a room or moving within a selected space in accordance with predetermined criteria.

In a certain embodiment, the control unit is programmed for controlling whether an activity signal is created by the infrared sensor within a predetermined time period. For example, the apparatus may be programmed to check, whether a person in a private home enters the bathroom in the morning. If this is not the case, it may be an indication of the person not feeling well or having difficulty to move from the bed to the bathroom. In this case, the control unit is configured to transmit an alarm signal to a central computer or predetermined terminal if no activity signal is created within the time period.

In order for the apparatus to detect activity in a selected area, for example confined to part of a room, the following embodiment is useful. In this case the infrared sensor is provided in a housing and the selected area is defined by an aperture in the housing and confined to a conical tunnel of sight from the infrared sensor. For example, the conical tunnel may be adjusted to match the area above a bed of a patient. Only if the patient rises from the bed or standing persons are in the area around the bed, the infrared sensor will be activated. Thus, the apparatus can be used to check whether the patient rises from the bed. If the patient is expected to stay in the bed due to a special disease, the detection that the patient rises may be used for sending an alarm to medical personnel. If the patient is expected to rise from the bed in the morning, an alarm may be sent, if this does not happen. Thus, depending on the programming of the apparatus or of a central computer connected to the apparatus the patient may get assistance in dependence of the actual need and circumstances.

The cross section of the conical tunnel may depend on the shape of the aperture. For example, the conical tunnel may be circular, elliptical, oval, rectangular of have any other advantageous shape depending on the selected area intended to be controlled.

In order to have easy means for adjusting the tunnel shape, the housing comprises a cover with a plurality of apertures and comprises fastening means for fastening the cover in different preselected orientations. Each preselected orientation implies a positioning of one selected of a plurality of apertures in front of the infrared sensor, the different apertures defining different tunnels of sight. For example, the cover may have four apertures and can be fastened in four different orientations. For example, depending on the aperture, the tunnel covers 1) only the area of the bed, 2) the area of the bed and the area closely around the bed, 3) the area above the bed, or 4) the entire bed room. By simply changing the orientation of the cover, the apparatus can be adjusted to the specific use at the area of interest.

In order to adjust the angular spread of the conical tunnel, the different apertures may be covered by different lenses, preferably Fresnel lenses. The lenses focus the infrared radiation from a selected area onto the infrared sensor. Depending on the selection of lens in front of the infrared sensor, different selected areas are controlled. For example, the first lens may be selected for an apparatus controlling the area above the bed in the bedroom, the second may be selected for a second apparatus checking the activity in the bathroom, the third may be selected for a third apparatus checking the activity in front of the cooking area in the kitchen, and the fourth lens may be a wide angle lens for a fourth apparatus checking activity in the entire living room.

As an alternative or in addition to the use of different lenses in the apertures, the apertures may be covered with lenses having different viewing angles. These different viewing angles can be accomplished by providing the apertures or the lenses or both with different angles relative to the infrared sensor. Thus, the viewing angle would be dependent on the orientation of the cover.

The apparatus according to the invention can as a supplement have a smoke sensor and be configured for transmitting a fire alarm to a central computer or another predetermined terminal in case of smoke detection.

Also, the electronic communication apparatus may also comprises an additional, infrared sensor for measuring infrared radiation from an infrared source having a temperature exceeding a preset temperature level. The preset level is set to above 40°C in order to differentiate between a person and a different heat source, for example a lit cigarette. In order to differentiate even further, the level may be set to above 70°C or above 100°C, for example above 200°C or even above 250°C in order to check on a cooking plate in a kitchen whether the cooking plate has not been turned off by mistake. The apparatus may then be configured, in case that the measured temperature exceeds the preset temperature level, to sound an alarm in order to alert the person who has forgotten to turn of the cooking plate and/or to send an alarm to the central computer or other predetermined terminal for help. The temperature of the infrared source can be determined by computerized evaluation of the frequency spectrum of the infrared radiation from the source.

In a further embodiment, the control unit in the apparatus may be programmed by signals from the central computer. Optionally, in order to save power on the general basis, the control unit is configured not to be in a programmable state all the time. Instead, the control unit is programmed for periodically changing from a power consumption minimizing mode to an alert mode, wherein the control unit is capable for being programmed by a digital programming signal from a central computer only if receiving a trigger signal from the central computer during the alert mode. Thus, the triggering signal will only influence the control unit when it is in the alert mode.

In order to be sure that a trigger signal from the central computer will reach the control unit when in an alert mode, the trigger signal has a time length longer than a time period between two alert modes. In this case, the trigger signal will always overlap at least one period and be received by the apparatus when switching to an alert mode.

When personnel in a hospital enter a room for a patient, it may be necessary that the personnel get assistance due to the actual circumstances, which may be a patient that has fallen out of the bed. In order to get assistance quickly without having to leave the patient, the device carried by the personnel is equipped with an alarm switch, the manual activation of which causes the device to transmit a help signal to the apparatus. In turn, the apparatus is programmed as a response to the help signal to forward the help signal in digital form to the central computer or to another predetermined terminal. When the signal reaches a central computer, the computer may check, whether there are further authorised personnel nearby the patient's room and may call these personnel to move to the room for assistance.

The patient may be equipped with a portable electronic device comprising a fall sensor capable of detecting a fall of the person carrying the fall sensor. A fall may induce an alarm signal transmitted to the apparatus and forwarded to the central computer or to another predetermined terminal.

The device with the fall sensor can advantageously be equipped with a motion sensor for sensing motion of the device and for creating a motion signal only in case of sensed motion. This is useful in the case, where an alarm only should be sent in case that a detected fall results in lack of motion, because an alarm may be only necessary if the patient is not getting on the feet again. For this sake, the apparatus may be configured upon receipt of a fall signal from the device to transmit a further request to the device for responding with the motion signal. Further, the apparatus is configured only to transmit the fall signal to the central computer or to another predetermined terminal in case that no motion signal is received.

In a further embodiment, the apparatus has digital data storage capabilities and is configured for receiving and storing patient related information. For example, the patient may be equipped with a temperature responsive transmitter as disclosed in US patent No. 4,844,076 by Lesho et al or a glucose sensor as disclosed US patent application No. 2005/267550 by Hess et al., and the apparatus may receive measured data from these sensors. The apparatus may, thus, store data from sensors that transmit physiological parameters from the patient, for example blood pressure, temperature and/or heart rate. Such stored data may optionally be transmitted to a central computer.

As a first alternative, the parameters may be analysed by a program in the control unit for determining alarm conditions in accordance with predetermined criteria. Such alarm conditions may cause the apparatus to submit a digital message to the central computer or another predetermined terminal.

As a second alternative, the apparatus may be programmed to receive the data from the sensors periodically, for example every half hour.

As an additional option, the sensor may be programmed to evaluate the sensed physiological parameters and programmed to submit an alarm to the apparatus only when the sensed physiological parameters are not within predetermined ranges. Only in the case when such an alarm is received, the apparatus forwards submits an alarm to the central computer.

Also, the parameters may be stored for later transmission to the computer of a doctor or nurse visiting the patient. In this way, the apparatus may serve as a monitoring and data storing part of a patient journal. For example, the apparatus may be programmed to communicate with the mobile computer, for example a PDA (Personal Digital Assistant), from the doctor or nurse for data exchange. The apparatus may submit accumulated physiological parameters and other data related to the patient to the mobile computer of the doctor and may in turn receive data for storing in the apparatus as part of the patient journal, for example evaluation data and comments from the doctor. In this way, the patient journal is always available at the site of the patient. The apparatus may also exchange relevant patient journal data with the central computer, such that the central computer is always updated with respect to the patient journal. In addition, the central computer may submit data from the patient journal to the apparatus in order to make relevant information available locally.

The apparatus according to the invention may be used for tracing personnel in a hospital. A plurality of such apparatus can, thus, be mounted on corridors, laboratories, rooms, etc., and may register any responding portable device that passes through the magnetic near field. The registration data are then transmitted to the central computer, which stores the data in a database. In case that the location of a certain person with a specific portable device is requested, the computer may on the basis of the data from the apparatus remit the location to the requester. This is either done by identifying the apparatus ID that did correspond to the portable device at the latest, in which case the response message may be "Person X registered at Apparatus 1522 at time 7.13 hrs", or the position as such may be found in case that the central computer has a list of the locations of the different apparatuses in the hospital, in which case the response message may be "Person X registered in building 22, 2^{nd} floor, corridor D" at time 7.13 hrs".

### Description of the Drawing

The invention will be explained in greater detail with reference to the drawing, where
FIG. 1 illustrates a first embodiment of an apparatus according to the invention,
FIG. 2 illustrates a housing with lenses and different angled apertures.

### Detailed Description of the Invention

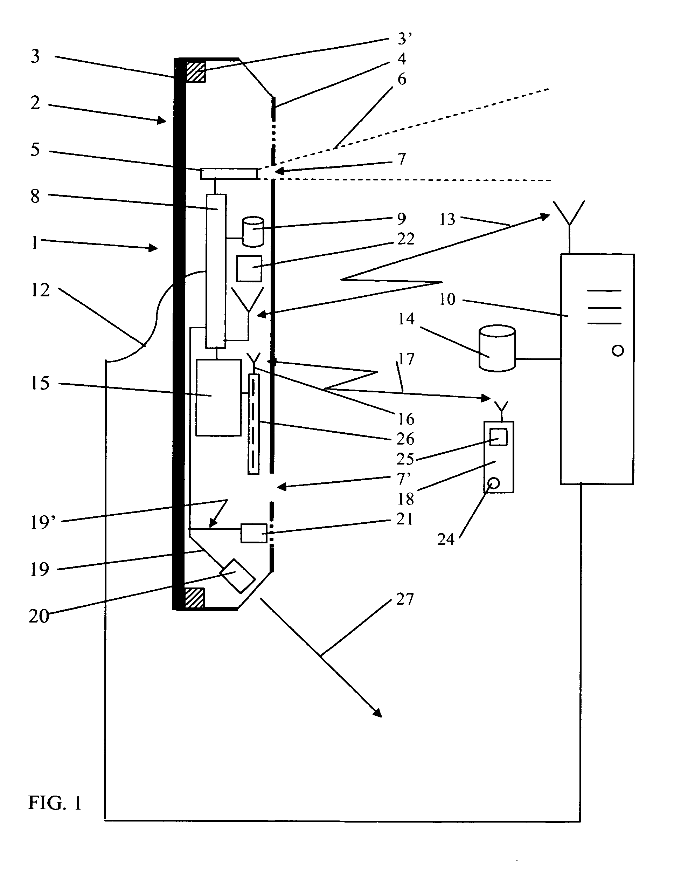

FIG. 1 shows an apparatus to be used according to the invention. The apparatus 1 has a housing 2 comprising a base 3 for fastening the device to a wall or ceiling and a cover 4 fastened to the base 3. Inside the housing 2 is arranged an infrared sensor 5 for measuring infrared radiation in a selected area in front the apparatus. The selected area is defined by a conical tunnel-of-sight 6 which is defined by an aperture 7 in the cover 4. The cover 4 may be fastened with fastening means 3' to the base 3 in different orientations. For example, the cover 4 may be rotated 180 degrees relative to the base such that a second aperture 7' is positioned in front of the infrared sensor, which results in a different tunnel-of-sight due to the different size of the aperture 7' and the position of the aperture 7' relative to the infrared sensor 5 when placed in front of the infrared sensor 5.

The infrared sensor 5 is electronically connected to a control unit 8 containing a processor and a memory with database 9. The control unit 8 is electronically connected to a central computer 10 either through a wired connection 12 or through a wireless connection 13. The central computer 10 has a database 14 for storage of data.

Optionally, the tunnel of sight of the infrared sensor 5 is delimited by plates, for example plates in the vertical plane. In dependence on the desired tunnel of sight, these plates may be reflecting, for example partly reflecting.

The control unit 8 is connected to a magnetic near field transceiver 15 having an antenna 16, for emission of near field radiation 17 for communication with a portable device 18, such as a pager, correspondingly configured mobile telephone or badge.

One non-limiting example of an antenna 16 is a construction like a coax type cable 26 having insufficient screening such that the magnetic near field radiation leaks out of the cable. Another possibility is a mistuned dipole antenna.

Optionally, the apparatus comprises a further infrared sensor 20, in the following called pyro sensor, for measuring infrared radiation from high temperature source, such as a fire, overheated food or a cooking plate. The control unit 8 is electronically connected 19 to the pyro-sensor 20 for retrieving electronic signals in from the pyro-sensor. For example, the apparatus may be installed in a kitchen with a line of sight 27 of the pyro-sensor directed towards the cooking plates such that an overheating of the cooking plate would induce an alarm signal, in the pyro-sensor which is transmitted to the control unit 8, which then forwards a digital alarm signal to the central computer or to another pre-selected terminal (not shown). An example of an alarm in connection with an electric range is disclosed in European patent EP 1 485 652 by Holdgaard.

In case that the apparatus is installed such that the pyro sensor registers the overheating of a cooking plate or overheating and burning of food, the signal from the pyro sensor 20 and a signal from the infrared sensor 5 can be differentiated, for example by blocking the infrared radiation from the cooking plate to enter the infrared sensor 5. Alternatively, the temperature dependence of the infrared radiation can be used to electronically discriminate between the infrared radiation from a person and the infrared radiation from overheated or burning food.

Optionally, the apparatus 1 may comprise a smoke sensor 21 as part of a fire alarm feature of the apparatus 1. The control unit 8 is electronically connected 19' to the smoke sensor 21 for retrieving electronic signals from the smoke sensor 21.

The control unit 8 is configured to retrieve the signals from the infrared sensor 5, the transceiver 15, the wireless connection 13 or wired connection 12, the pyro-sensor 20 and the smoke sensor 21 and programmed to act in a predetermined way in dependence of the signal. For example, signals from the different units are transmitted to the central computer 10. Alternatively, the signals are stored in the database 9 for later extraction, and only in case of alarms, corresponding data are transmitted to the central computer 10 and/or other pre-selected terminal. Preferably, power for the apparatus 1 is provided by a battery 22.

In addition to the illustrated elements, the apparatus may also comprise an on/off switch and control lamps, preferably low power light emitting diodes (LED).

The portable device 18 is equipped with a display 25 and an alarm switch 24, the manual activation of which causes the device to transmit an alarm signal to the apparatus 1. In turn, the control unit 8 is programmed as a response to the alarm signal to forward the alarm signal in digital form to the central computer 10 or to another predetermined terminal. When the signal reaches the central computer 10, the computer 10 may check, whether there is an alarm routine to be run as a response to the alarm signal. For example, the alarm switch 24 may have been activated for calling help, and the computer recognises the alarm signal as a help request associated with a help routine, causing the call of further authorised personnel nearby the patient's room to move to the room for assistance.

The cover 4 is illustrated in FIG. 1 with two different apertures 7, 7' for changing the tunnel-of-sight 6 by mounting the cover 4 turned 180 degrees. However, the cover may have more than 2 apertures 7, 7', for example 4 apertures, which are varied in front of the infrared sensor 21 by 90 degrees rotation of the cover.

As an alternative, the apparatus 1 may be constructed with adjustable apertures, for example comprising horizontally and vertically adjustable screening plates.

The apertures 7, 7' may be covered with lenses, for example Fresnel lenses for focusing infrared radiation from a selected area or part of the area onto the infrared sensor 21. Optionally, the Fresnel lens reflecting infrared radiation to the lens may be configured to have different parts of the lens covering different parts of the selected area in order to measure movement within the selected area.

In addition to the form and size of the apertures, or alternatively, the lenses may be different. The advantage of such variation of apertures and/or lenses is the fact that the user after having purchased such an apparatus 1 can adjust the capture of infrared radiation by mounting the cover 4 in different orientations. For the different apertures and/or lenses, the selected area is different.

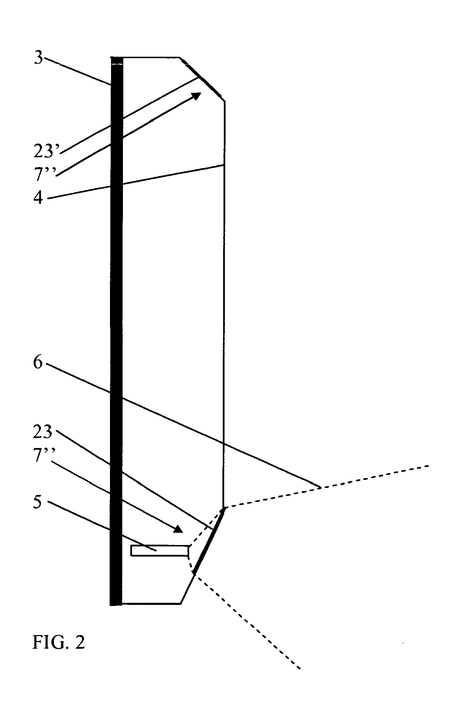

In order to vary the selected area, the apertures with lenses may have varying angle with respect to the base and the infrared sensor 5. This is illustrated in FIG. 2, where only the base 2, the cover 4, the infrared sensor 5 and the lenses 23, 23' in the apertures 7" are shown, even though all of the other units from FIG. 1 or a selection thereof are included.

## Claims

1. Use of an electronic communication apparatus for checking authorisation of personnel or visitors in a hospital the communication apparatus comprising
- an infrared sensor for detection of infrared radiation in a selected area in the vicinity of the detector, the infrared sensor being configured to create an activity signal upon detection of movement of an infrared radiation source in the area, the activity signal causing an activity mode;
- a transceiver for wireless near field magnetic communication with a portable electronic device,
- a control unit with a digital processor electronically connected to the infrared sensor and to the transceiver,
wherein the control unit is programmed for causing the transceiver to transmit a wireless near field request signal only in the activity mode as a request for receiving a wireless response signal with an ID from a portable electronic device.

2. Use according to claim 1, wherein the control unit is configured, upon receiving the ID of the portable device by the transceiver, to submit the ID of the portable electronic device as a digital signal to a central computer.

3. Use according to claim 1, wherein the control unit is provided with an ID list and is configured to compare the received ID with the ID list, and wherein the control unit is configured only to automatically submit an alarm or the ID of the portable electronic device or both as a digital signal to a central computer, if the received ID is not found in the ID list.

4. Use according to any preceding claim, wherein the control unit is configured upon lack of receiving an ID of a portable device to automatically submit a digital alarm signal to a central computer.

5. Use according to any preceding claim, wherein the control unit is programmed for controlling whether an activity signal is created by the infrared sensor within a predetermined time period, and wherein the control unit is configured to transmit an alarm signal to a central computer if no activity signal is created.

6. Use according to any preceding claim, wherein control unit is programmed for
- minimizing the power consumption of the electronic communication apparatus, including causing a sleep mode of the transceiver when no activity signal is received from the infrared sensor,
- changing the sleep mode into an activity mode for a predetermined period of time, as a response to the activity signal from the infrared sensor,
- causing the transceiver to transmit the request for receiving signal only when in the activity mode.

7. Use according to any preceding claim, wherein the infrared sensor is provided in a housing and the selected area is defined by an aperture in the housing and confined to a conical tunnel of sight from the infrared sensor.

8. Use according claim 7, wherein the housing comprises a cover with a plurality of apertures and comprises fastening means for fastening the cover in different preselected orientations, each preselected orientation implying a positioning of one selected of a plurality of apertures in front of the infrared sensor, the different apertures defining different tunnels of sight.

9. Use according claim 7, wherein the different apertures are covered with different Fresnel lenses for focusing the infrared radiation from a selected area onto the infrared sensor, the different Fresnel lenses being configured for different selected areas in dependence of the selected aperture.

10. Use according claim 9, wherein the apertures are covered with Fresnel lenses for focusing the infrared radiation from a selected area onto the infrared sensor, wherein the angle of the Fresnel lens in the selected aperture relative to the infrared sensor is dependent on the orientation of the cover.

11. Use according to any preceding claim, wherein the apparatus also comprises a smoke sensor and is configured for transmitting a fire alarm to a central computer in case of smoke detection.

12. Use according to any preceding claim, wherein the apparatus also comprises an additional, infrared sensor for measuring infrared radiation from an infrared source having a temperature exceeding a preset temperature level, the preset temperature level being above 40°C, wherein the apparatus is configured for sending an alarm if the measured temperature exceeds the preset temperature level.

13. Use according to any preceding claim, wherein the control unit is programmed for periodically changing from a power consumption minimizing mode to an alert mode, wherein the control unit is capable for being programmed by a digital programming signal from a central computer only if receiving a trigger signal from the central computer during the alert mode.

14. Use according to claim 11 wherein the electronic communication apparatus is provided in combination with a central computer connected to the apparatus, wherein the central computer is configured for sending the trigger signal to the apparatus, the trigger signal having a time length longer than a time period between two alert modes.

15. Use according to any one of the claims 1-11 wherein the electronic communication apparatus is provided in combination with a portable electronic device, wherein the device is provided with an alarm switch, the manual activation of which causes the device to transmit a help signal to the apparatus, and wherein the apparatus is programmed as a response to the help signal to forward the help signal in digital form to the central computer.

16. Use according to any one of the claims 1-11 wherein the electronic communication apparatus is provided in combination with a portable electronic device, wherein the device comprises a fall sensor capable of detecting a fall of a person carrying the fall sensor, wherein the device upon detection of a fall is configured for transmitting a fall signal to the apparatus, and wherein the apparatus is configured as a response to the fall signal to forward the fall signal in digital form to the central computer.

17. Use of a combination according to claim 14, wherein the device has a motion sensor for sensing motion of the device and for creating a motion signal only in case of sensed motion, wherein the apparatus is configured upon receipt of a fall signal from the device to transmit a further request to the device for responding with the motion signal, and wherein the apparatus is configured only to transmit the fall signal to the central computer in case that no motion signal is received.

## Patentansprüche

1. Verwendung eines elektronischen Kommunikationsgeräts zur Überprüfung der Zugangsberechtigung von Personal oder Besuchern in einem Krankenhaus, wobei das Kommunikationsgerät folgendes aufweist:
- einen Infrarotsensor zum Ermitteln von Infrarotstrahlung in einem ausgewählten Bereich in der Nähe des Detektors, wobei der Infrarotsensor zum Erzeugen eines Aktivitätssignals nach der Ermittlung einer Bewegung einer Infrarotstrahlungsquelle im Bereich konfiguriert ist, wobei das Aktivitätssignal einen Aktivitätsmodus veranläßt,
- einen Sendeempfänger für drahtlose, nahfeld-magnetische Kommunikation mit einer tragbaren elektronischen Einrichtung,
- eine Steuereinheit mit einem mit dem Infrarotsensor und dem Sendeempfänger elektronisch verbundenen Digitalprozessor,
wobei die Steuereinheit für die Veranlassung von dem Übermitteln eines drahtlosen Nahfeld-Anforderungssignals nur im Aktivitätsmodus als Anforderung auf den Empfang eines drahtlosen Anwortsignals mit einer ID aus einer tragbaren elektronischen Einrichtung programmiert ist.

2. Verwendung nach Anspruch 1, wobei die Steuereinheit, nach dem Empfang des Sendeempfängers von der ID der tragbaren Einrichtung, für die Abgabe der ID der tragbaren elektronischen Einrichtung als ein digitales Signal an einen zentralen Computer konfiguriert ist.

3. Verwendung nach Anspruch 1, wobei die Steuereinheit eine ID-Liste aufweist und für das Vergleichen der empfangenen ID mit der ID-Liste konfiguriert ist, und wobei die Steuereinheit nur für die automatische Abgabe eines Alarms oder der ID der tragbaren elektronischen Einrichtung oder beide als ein digitales Signal an einen zentralen Computer, wenn die empfangene ID nicht in der ID-Liste gefunden wird, konfiguriert ist.

4. Verwendung nach irgendeinem der vorhergehenden Ansprüche, wobei die Steuereinheit für automatische Abgabe eines digitalen Alarmsignals an einen zentralen Computer nach einem fehlenden Empfang von einer ID einer tragbaren Einrichtung konfiguriert ist.

5. Verwendung nach irgendeinem der vorhergehenden Ansprüche, wobei die Steuereinheit für die Überprüfung einer eventuellen Erzeugung des Infrarotsensors von einem Aktivitätssignal innerhalb eines vorbestimmten Zeitraums programmiert ist, und wobei die Steuereinheit für das Übermitteln eines Alarmsignals an den zentralen Computer, wenn kein Aktivitätssignal erzeugt wird, konfiguriert ist.

6. Verwendung nach irgendeinem der vorhergehenden Ansprüche, wobei die Steuereinheit für folgendes programmiert ist:
- Minimierung von dem Energieverbrauch des elektronischen Kommunikationsgeräts, einschließlich Veranlassung von einem Schlafmodus des Sendeempfängers, wenn kein Aktivitätssignal aus dem Infrarotsensor empfangen wird,
- Änderung vom Schlafmodus zu einem Aktivitätsmodus für einen vorbestimmten Zeitraum als Antwort auf einem Aktivitätssignal aus dem Infrarotsensor,
- Veranlassung des Sendeempfängers nur im Aktivitätmodus zum Übermitteln der Anforderung auf dem Empfangen eines Signals.

7. Verwendung nach irgendeinem der vorhergehenden Ansprüche, wobei der Infrarotsensor in einem Gehäuse angeordnet ist, und der gewählte Bereich durch eine Öffnung im Gehäuse definiert ist und auf einen konischen Sichttunnel von dem Infrarotsensor beschränkt ist.

8. Verwendung nach Anspruch 7, wobei das Gehäuse eine Abdeckung mit einer Vielzahl von Öffnungen umfasst und Befestigungsmittel zur Befestigung der Abdeckung in unterschiedlichen vorgewählten Ausrichtungen aufweist, wobei die vorgewählten Ausrichtungen jeweils eine Positionierung einer aus der Vielzahl von Öffnungen ausgewählten Öffnung vor dem Infrarotsensor implizieren, und die unterschiedlichen Öffnungen unterschiedliche Sichttunnellen definieren.

9. Verwendung nach Anspruch 7, wobei die unterschiedlichen Öffnungen zur Scharfstellung der Infrarotstrahlung aus einem ausgewählten Bereich auf den Infrarotsensor durch unterschiedliche Fresnel-Linsen gedeckt sind, wobei die unterschiedlichen Fresnel-Linsen für unterschiedliche ausgewählten Bereiche in Abhängigkeit von der ausgewählten Öffnung konfiguriert sind.

10. Verwendung nach Anspruch 9, wobei die Öffnungen zur Scharfstellung der Infrarotstrahlung aus einem ausgewählten Bereich auf den Infrarotsensor durch Fresnel-Linsen gedeckt werden, wobei der Winkel der Fresnel-Linse in einer ausgewählten Öffnung relativ zum Infrarotsensor von der Ausrichtung der Abdeckung abhängig ist.

11. Verwendung nach irgendeinem der vorhergehenden Ansprüche, wobei das Gerät auch einen Rauchsensor umfasst und im Falle einer Rauchermittlung für das Übermitteln einer Brandmeldung an einen zentralen Computer konfiguriert ist.

12. Verwendung nach irgendeinem der vorhergehenden Ansprüche, wobei das Gerät auch einen weiteren Infrarotsensor zum Messen der Infrarotstrahlung aus einer Infrarotquelle mit einer eine vorgegebene Temperaturebene überschreitende Temperatur umfasst, wobei die vorgegebene Temperaturebene mehr als 40°C beträgt, wobei das Gerät zum Senden eines Alarms, wenn die gemessene Temperatur die vorgegebene Temperaturebene überschreitet, konfiguriert ist.

13. Verwendung nach irgendeinem der vorhergehenden Ansprüche, wobei die Steuereinheit für periodische Änderung von einem Energieverbrauchsminimierungsmodus zu einem Bereitschaftsmodus programmiert ist, wobei die Steuereinheit durch ein digitales Programmierungssignal aus einem zentralen Computer nur beim Empfangen eines Auslösesignals aus der zentralen Computer während dem Bereitschaftsmodus programmierbar ist.

14. Verwendung nach Anspruch 11, wobei das elektronische Kommunikationsgerät in Kombination mit einem zentralen, mit dem Gerät verbundenen Computer angeordnet ist, wobei der zentrale Computer für das Senden des Auslösesignals an das Gerät konfiguriert ist, konfiguriert ist, wobei das Auslösesignal eine Dauer von mehr als einem Zeitraum zwischen zwei Bereitschaftsmodi beträgt.

15. Verwendung nach irgendeinem der Ansprüche 1-11, wobei das elektronische Kommunikationsgerät in Kombination mit einer tragbaren elektronischen Einrichtung angeordnet ist, wobei die Einrichtung einen Alarmschalter aufweist, dessen manuelle Betätigung das Übermitteln eines Hilfssignals von der Einrichtung an das Gerät veranlässt, und wobei das Gerät als Antwort auf dem Hilfssignal zur Weiterleitung des Hilfssignals in digitaler Form an den zentralen Computer programmiert ist.

16. Verwendung nach irgendeinem der Ansprüche 1-11, wobei das elektronische Kommunikationsgerät in Kombination mit einer tragbaren elektronischen Einrichtung angeordnet ist, wobei die Einrichtung einen Fallsensor umfasst, der einen Sturtz einer den Fallsensor tragenden Person ermitteln kann, wobei die Einrichtung nach der Ermittlung eines Sturtzes für das Übermitteln eines Fallsignals an das Gerät konfiguriert ist, und wobei das Gerät als eine Antwort auf dem Fallsignal zur Weiterleitung des Fallsignals in digitaler Form an den zentralen Computer konfiguriert ist.

17. Verwendung einer Kombination nach Anspruch 14, wobei die Einrichtung einen Bewegungssensor zur Erkennung einer Bewegung der Einrichtung und zum Erzeugen eines Bewegungssignals nur bei der Erkennung einer Bewegung aufweist, wobei das Gerät nach dem Empfang eines Fallsignals aus der Einrichtung für das Übermitteln einer weiteren Anforderung an die Einrichtung auf Beantwortung mit dem Bewegungssignal konfiguriert ist, und wobei das Gerät nur für das Übermitteln des Fallsignals an einen zentralen Computer, wenn kein Bewegungssignal empfangen wird, konfiguriert ist.

## Revendications

1. Appareil de communication électronique comprenant
- un capteur à infrarouges pour la détection d'un rayonnement infrarouge dans une région sélectionnée dans la proximité du capteur, le capteur à infrarouges étant configuré pour créer un signal d'activité lors d'une détection d'un mouvement d'une source de rayonnement infrarouge dans la région
- un récepteur pour la communication sans fil avec un dispositif électronique portable,
- une unité de contrôle avec un processeur numérique reliée au capteur à infrarouges et au récepteur,
dans lequel l'unité de contrôle est programmée pour entraîner que le récepteur transmette un signal de demande sans fil comme une demande de recevoir un signal de réponse sans fil avec une ID d'un appareil électronique portable,
**caractérisé en ce que** le récepteur est un récepteur pour une communication de champ magnétique proche sans fil avec le dispositif portable, et le signal sans fil venant de l'unité de contrôle constitue un signal de demande de champ magnétique proche et sans fil.

2. Appareil de communication électronique selon la revendication 1, dans lequel l'unité de contrôle est configurée, lors de la réception de l'ID du dispositif portable par le récepteur, pour soumettre l'ID de l'appareil électronique portable comme un signal numérique à un ordinateur central.

3. Appareil de communication électronique selon la revendication 1, dans lequel l'unité de contrôle est munie d'une liste d'ID et configurée pour comparer l'ID reçue avec la liste d'ID, et dans lequel l'unité de contrôle est configurée seulement pour émettre automatiquement une alarme ou l'ID du dispositif électronique portable ou les deux comme un signal numérique à un ordinateur central si l'ID reçue n'est pas trouvée sur la liste d'ID.

4. Appareil de communication électronique selon l'une quelconque des revendications précédentes, dans lequel l'unité de contrôle est configurée, lors d'un manque de réception d'une ID d'un dispositif portable, pour soumettre automatiquement un signal d'alarme numérique à un ordinateur central.

5. Appareil de communication électronique selon l'une quelconque des revendications précédentes, dans lequel l'unité de contrôle est programmée pour contrôler si le signal d'activité est créé par le capteur à infrarouges dans un délai prédéterminé, et dans lequel l'unité de contrôle est configurée pour transmettre un signal d'alarme à un ordinateur central si aucun signal d'activité est créé.

6. Appareil de communication électronique selon l'une quelconque des revendications précédentes, dans lequel
l'unité de contrôle est programmée pour
- minimiser la consommation d'énergie de l'appareil de communication électronique, y compris activant un mode veille du récepteur si aucun signal d'activité n'est reçu du capteur à infrarouges,
- modifier le mode veille à un mode d'activité dans un délai prédéterminé, comme une réponse au signal d'activité du capteur à infrarouges,
- mener le récepteur à transmettre la demande de recevoir un signal seulement en mode d'activité.

7. Appareil de communication électronique selon l'une quelconque des revendications précédentes, dans lequel le capteur à infrarouges est prévu dans un boîtier et la région sélectionnée est définie par une ouverture dans le boîtier et confinée à une vue en forme de tunnel conique venant du capteur à infrarouges.

8. Appareil de communication électronique selon la revendication 7, dans lequel le boîtier comprend un couvercle ayant plusieurs ouvertures et comprend un moyen de fixation pour fixer le couvercle dans différentes orientations présélectionnées, chaque orientation présélectionnée comprend un positionnement sélectionné parmi plusieurs ouvertures devant le capteur à infrarouges, les différentes ouvertures définissent des différents tunnels de vue.

9. Appareil de communication électronique selon la revendication 7, dans lequel les différentes ouvertures sont couvertes de différentes lentilles infrarouge de Fresnel pour focaliser le rayonnement rouge d'une région sélectionnée au capteur à infrarouges, les différentes lentilles de Fresnel sont configurées pour différentes régions sélectionnées en fonction de l'ouverture selectionnée.

10. Appareil de communication électronique selon la revendication 9, dans lequel les ouvertures sont couvertes de lentilles de Fresnel pour focaliser le rayonnement infrarouge d'une région sélectionnée au capteur à infrarouges, dans lequel l'angle des lentilles de Fresnel dans l'ouverture sélectionnée par rapport au capteur à infrarouges dépend de l'orientation du couvercle.

11. Appareil de communication électronique selon l'une quelconque des revendications précédentes, dans lequel l'appareil comprend également un capteur de fumée et est configuré pour transmettre une alarme d'incendie à un ordinateur central en cas de détection de fumée.

12. Appareil de communication électronique selon l'une quelconque des revendications précédentes, dans lequel l'appareil comprend également un capteur à infrarouges supplémentaire pour mésurer le rayonnement infrarouge d'une source infrarouge ayant une température dépassant un niveau de température prédéfini, le niveau de température prédéfini est supérieur à 40°C, dans lequel l'appareil est configuré pour envoyer une alarme si la température mesurée dépasse le niveau de température prédéfini.

13. Appareil de communication électronique selon l'une quelconque des revendications précédentes, dans lequel l'unité de contrôle est programmée pour passer périodiquement d'un mode de réduction de la consommation électrique à un mode d'alerte, dans lequel l'unité de contrôle peut être programmée par un signal numérique de programmation d'un ordinateur central seulement si un signal de déclenchement est reçu de l'ordinateur central en mode d'alerte.

14. Appareil de communication électronique selon la revendication 11 en combinaison avec un ordinateur central relié à l'appareil, dans lequel l'ordinateur central est configuré pour envoyer le signal de déclenchement à l'appareil, le signal de déclenchement comprend une durée de temps plus longue que la durée de la période de temps entre deux modes d'alerte.

15. Appareil de communication électronique selon l'une quelconque des revendications 1-11 en combinaison avec un dispositif électronique portable, dans lequel le dispositif est réalisé avec un interrupteur d'alarme dont le déclenchement manuel fait que le dispositif transmet un signal d'aide à l'appareil, et dans lequel l'appareil est programmé comme une réponse au signal d'aide pour transmettre le signal d'aide en forme numérique à l'ordinateur central.

16. Appareil de communication électronique selon l'une quelconque des revendications 1-11 en combinaison avec un dispositif électronique portable, dans lequel le dispositif comprend un capteur de chute capable de détecter une chute d'une personne portant le capteur de chute, dans lequel le dispositif est configuré pour transmettre un signal de chute à l'appareil après une détection d'une chute, et dans lequel l'appareil est configuré pour transmettre le signal de chute en forme numérique à l'ordinateur central comme une réponse à un signal de chute.

17. Une combinaison selon la revendication 14, dans lequel le dispositif comprend un capteur de mouvement pour détecter le mouvement du dispositif et pour créer un signal de mouvement seulement en cas d'un mouvement détecté, dans lequel l'appareil est configuré, après la réception d'un signal de chute du dispositif, pour transmettre une demande en plus au dispositif pour répondre avec le signal de mouvement, et dans lequel l'appareil est configuré seulement pour transmettre le signal de chute à l'ordinateur central au cas où aucun signal de mouvement est reçu.
